## Europäisches Patentamt

(19) **European Patent Office**

**Office européen des brevets**

(11) Numéro de publication: **0 049 181**
**B1**

(12) **FASCICULE DE BREVET EUROPEEN**

(45) Date de publication du fascicule du brevet:
**08.08.84**

(21) Numéro de dépôt: **81401414.8**

(22) Date de dépôt: **11.09.81**

(51) Int. Cl.³: **A 61 K 39/108,** A 61 K 37/02,
A 61 K 45/05, C 12 P 21/00

(54) Nouvelles glycoprotéines hydrosolubles immunostimulantes, extraites de Klebsiella Pneumoniae, leur procédé d'obtention, leur application à titre de médicaments et les compositions les renfermant.

(30) Priorité: **19.09.80 FR 8020187**

(43) Date de publication de la demande:
**07.04.82 Bulletin 82/14**

(45) Mention de la délivrance du brevet:
**08.08.84 Bulletin 84/32**

(84) Etats contractants désignés:
**AT BE CH DE GB IT LI LU NL SE**

(56) Documents cités:
**FR - A - 2 043 475**
**FR - A - 2 088 112**
**FR - A - 2 171 907**
**FR - A - 2 253 499**
**FR - A - 2 396 020**
**FR - A - 2 462 477**

**La Restauration Immunitaire de la Théorie à la Pratique -**
**BIOSTIN - Publication des Laboratoires Cassenne 3,**
**square.Desaix 75015 PARIS**

**Le dossier contient des informations techniques**
**présentées postérieurement au dépôt de la demande et**
**ne figurant pas dans le présent fascicule.**

(73) Titulaire: **ROUSSEL-UCLAF, 35, boulevard des Invalides,**
**F-75007 Paris (FR)**

(72) Inventeur: **Zalisz, René, Résidence Pont**
**Petit-BâT. 3A 19, rue de France,**
**F-95310-Saint-Ouen-L'aumone (FR)**
Inventeur: **Salles, Marie-France, 97, Avenue de Villiers,**
**F-75017-Paris (FR)**

(74) Mandataire: **Vieillefosse, Jean-Claude et al,**
**ROUSSEL-UCLAF Boîte postale no. 9 111, route de**
**Noisy, F-93230 Romainville (FR)**

## Description

La présente invention concerne de glycoprotéines hydrosolubles immunostimulantes extraites de Klebsiella pneumoniae, leur procédé d'obtention, leur application comme médicaments et les compositions les renfermant.

Un certain nombre de brevets français ont déjà décrit des glycoprotéines extraites de Klebsiella pneumoniae,il en est ainsi, par exemple, des brevets français No 2.043.475, 2.088.112, et 2.171.907.

La présente demande concerne des glycoprotéines plus précisément définies et a ainsi pour objet de nouvelles glycoprotéines hydrosolubles immunostimulantes extraites de Klebsiella pneumoniae, caractérisées en ce qu'elles renferment 20% à 35% de protéines, 50% à 70% d'oses neutres, 3% à 8% d'acide glucuronique et 1,5% à 3% d'osamines, et ont un poids moléculaire supérieur à 100.000 daltons.

On désigne par oses neutres, notamment, des hexoses neutres tels que le glucose, le mannose ou le galactose.

Le poids moléculaire des glycoprotéines de la présente demande a été estimé par ultra centrifugation; on a trouvé trois fractions de poids moléculaire supérieur à 100.000 daltons.

Les glycoprotéines de l'invention peuvent être extraites de différentes souches de Klebsiella pneumoniae; on retient cependant tout particuliérement celles qui proviennent de la souche déposée à l'Institut PASTEUR sous le numéro 52.145.

L'étude de la structure de ces glycoprotéines à l'aide de différentes techniques chimiques, notamment la réduction des résidus acides uroniques par le carbodiimide, la perméthylation, la dégradation uronique, l'oxydation périodique ou l'oxydation par l'oxyde de chrome, a permis de préciser leur composition.

Les glycoprotéines préférées selon l'invention sont celles pour lesquelles la fraction protéique est composée par environ 30% d'acides aminés acides. On entend par acides aminés acides des acides aminés tels que l'acide aspartique ou l'acide glutamique.

Les glycoprotéines préférées selon l'invention sont aussi celles pour lesquelles la fraction polysaccharidique renferme approximativement pour une molécule de glucose, de 1 à 1,5 molécule de mannose, de 0,2 à 0,6 molécule d'acide glucuronique et de 2 à 2,5 molécules de galactose.

L'invention a également pour objet un procédé d'obtention des nouvelles glycoprotéines hydrosolubles telles que définies ci-dessus, dans lequel l'on prépare un extrait de glycoprotéines tel que celui obtenu par mise en culture de germes de Klebsiella pneumoniae, lyse des germes, séchage, délipidation par extraction à l'aide de solvants des lipides, et ultrafiltration, ledit procédé étant caractérisé en ce que l'on traite la solution aqueuse de glycoprotéines ainsi obtenue à l'aide d'un sel d'ammonium quaternaire, obtient ainsi un précipité que l'on élimine,traite à froid le surnageant à l'aide d'un alcanol de faible poids moléculaire, obtient ainsi un nouveau précipité que l'on isole, redissout dans l'eau, dialyse le cas échéant puis, lyophilise.

On peut utiliser différentes solutions de glycoprotéines au départ du procédé; ces solutions sont obtenues par diafiltration de lysats de cultures de Klebsiella pneumoniae sur des membranes poreuses calibrées, capables de retenir les molécules d'un poids moléculaire égal ou supérieur à un poids moléculaire donné, qui est une constante pour ces membranes.

Les membranes utilisées sont, par exemple, les membranes vendues sous les noms de marque AMICON XM50, PM30 et UM2, mais on préfère utiliser des membranes dont le seuil de rétention est de 100.000 daltons, telles que les membranes commercialisées sous la désignation XM 100 ou H1 P100, par la société AMICON; les membranes tout particulièrement préférées permettent de retenir les molécules dont le poids moléculaire est supérieur à 300.000 daltons; celles-ci sont avantageusement constituées par les membranes commercialisées sous la désignation XM300 par les sociétés AMICON et ROMICON.

La diafiltration permet de sélectionner, en solution, des molécules dont le poids moléculaire est supérieur à un poids moléculaire donné, comme on l'a dit, par le choix de la membrane, en fonction du seuil de rétention désiré. Il est évident, pour l'homme de l'art, que l'utilisation d'autres solutions de mêmes caractéristiques, obtenues par d'autres moyens, comme par exemple par chromatographie sur gel polymérisé hydrophile, est tout à fait possible.

Préalablement à cette opération de sélection, le lysat peut, très avantageusement, étre délipidé et débar rassé de ses acides nucléiques.

Dans des conditions de réalisation tout particulièrement préférées du procédé selon l'invention, la solution de glycoprotéines de départ est abtenue selon le procédé décrit dans le second certificat d'addition n° 2.171.907 au brevet français n° 2.043.475, c'est-à-dire par mise en culture des germes (Klebsiella pneumoniae CIP 52145 identique à la souche déposée par la demanderesse le 29 Juin 1981 sous le numéro I-163 à l'Institut Pasteur à Paris), lyse des germes, séchage (lyophilisation par exemple), extraction à l'aide de solvants des lipides, puis ultracentrifugation et séchage, par exemple par lyophilisation.

L'ammonium quaternaire que l'on utilise pour traiter la solution de glycoprotéines de départ peut être, par exemple, le chlorure de pyridyl-cétyl-ammonium, mais tout particulièrement, le bromure de triméthyl cétyl ammonium ou Cétavlon®.

0 049 181

Après traitement par l'ammonium quaternaire, le précipité obtenu peut être séparé du surnageant par des procédés classiques, tels que la décantation ou la filtration, mais on préfère le séparer par centrifugation.

La solution correspondant au surnageant est alors traitée à froid, aux environs de +4°C, à l'aide d'un alcanol de faible poids moléculaire tel que le méthanol, l'éthanol, le n-propanol ou l'isopropanol. On utilise de préférence l'éthanol. Les résultats les plus intéressants sont obtenus par l'utilisation de six volumes d'éthanol pour un volume de solution saline, pendant une nuit, à la température de +4°C.

Le précipité est redissous dans l'eau et dialysé pour le purifier. La dialyse est effectuée à l'aide de cellules de type classique obturées par des membranes, par exemple, de collodion ou de cellulose. On retient, notamment, les cellules dont la membrane est en cellulose régénérée, à diamètre moyen des pores égal à environ 24 Å, retenant les substances dont le poids moléculaire est supérieur à 12 à 14 000 daltons. Parmi les cellules à dialyse, on peut noter tout particulièrement les tubes VISKING®.

Cette dialyse permet d'éliminer de la solution de glycoprotéines, les impuretés de faible poids moléculaire restantes, comme les traces d'alcanol.

On peut évidemment obtenir les glycoprotéines de l'invention, un peu moins pures, en n'effectuant pas l'étape de dialyse.

La lyophilisation est effectuée de façon classique, par exemple dans des ensembles congélateur-sublimeur de taille moyenne comme les modèles SMU ou SMRG commercialisés par la Société Usifroid, des lyophilisateurs de grande taille comme par exemple, l'ensemble formé par un congélateur CA1 et un sublimeur SMIRS®, tous deux commercialisés par Usifroid. Des modèles plus petits, de laboratoire, peuvent aussi être utilisés, ainsi que ceux commercialisés par d'autres sociétés, telle la société Sérail.

Dans des conditions préférentielles de mise en œuvre, le procédé ci-dessus décrit est caractérisé en ce que:

— l'ammonium quaternaire utilisé est le bromure de cétyltriméthyl-ammonium;
— l'on isole par centrifugation le surnageant;
— l'alcanol de faible poids moléculaire est l'éthanol.

La présente demande a également pour objet les glycoprotéines telles qu'obtenues par le procédé selon l'invention.

Les glycoprotéines, objet de la présente invention, possèdent de très intéressantes propriétés pharmacologiques; elles sont douées, notamment, de remarquables propriétés immunostimulantes, anisi que d'une très bonne tolérance.

Ces propriétés sont illustrées plus loin dans la partie expérimentale.

Ces propriétés justifient l'utilisation des glycoprotéines de la présente demande, à titre de médicaments. La présente demande a ainsi également pour objet l'application, à titre de médicaments, des glycoprotéines telles que définies ci-dessus.

Parmi les médicaments de l'invention, on retient particulièrement ceux, caractérisés en ce qu'ils sont constitués par les glycoprotéines telles qu'obtenues par le procédé de l'invention.

Les médicaments de l'invention trouvent, par exemple, leur emploi dans le traitement ou la prévention, chez l'homme et l'animal, des maladies infectieuses causées par les bactéries ou les virus, dans le traitement des maladies à parasites, des toxi-infections, dans le traitement des infections posthospita-lières et postchirurgicales.

La dose usuelle, variable selon le produit utilisé, le sujet traité et l'affection en cause, peut-être par exemple, chez l'homme, de 1 mg à 15 mg par jour, par voie orale, de 1 mg à 15 mg par jour, par voie rectale et de 0,25 mg à 5 mg par jour, par voie parentérale.

L'invention a enfin pour objet les compositions pharmaceutiques qui renferment les glycoprotéines de la présente demande, à titre de principe actif.

A titre de médicaments, les glycoprotéines de la présente demande peuvent être administrées par les voies digestive, parentérale et locale.

Les compositions pharmaceutiques correspondantes peuvent être, par exemple, solides ou liquides et se présenter sous les formes pharmaceutiques couramment utilisées en médecine humaine, comme par exemple, les comprimés, simples ou dragéifiés, les gélules, les granulés, les solutions, les sirops, les suppositoires, les préparations injectables lyophilisées ou non, les ovules, les crèmes, les pommades, les lotions, les gouttes, les collyres, les aérosols, elles sont préparées selon les méthodes usuelles. Le ou les principes actifs peuvent y être incorporés à des excipients habituellement employés dans ces compositions pharmaceutiques, tels que le talc, la gomme arabique, le lactose, l'amidon, le stéarate de magnésium, le beurre de cacao, les véhicules aqueux ou non, les corps gras d'origine animale ou végétale, les dérivés paraffiniques, les glycols, les divers agents mouillants, dispersants ou émulsifiants, les conservateurs.

Il va être donné maintenant, à titre non limitatif, des exemples de mise en œuvre de l'invention.

3

## Exemple 1:

20 g de produit tel qu'obtenu à l'exemple n° 1 du brevet français n° 2.171.907, sont dissous dans deux litres d'eau permutée.

On ajoute, lentement, environ 1,6 l de Cetavlon® à 3%, l'ensemble est agité pendant une heure, puis centrifugé à 10.000 t/mn, pendant 15 minutes. On élimine le précipité, ajoute ensuite au surnageant isolé 3 l d'éthanol à 95°, en 15 minutes. Après une heure d'agitation, on centrifuge à 10.000 t/mn, pendant 15 minutes. Le surnageant ainsi obtenu est éliminé et le précipité est redissous dans 1 l d'eau puis dialysé pendant 48 heures dans des tubes Visking® contre de l'eau permutée à +4°C. Au terme de cette dialyse, la solution est lyophilisée. On obtient 7,12 g des glycoprotéines cherchées.

## Exemple 2:

20 g de produit tel qu'obtenu à l'exemple 1 du brevet français n° 2.171.907, sont dissous dans 1 l d'eau permutée. On ajoute sous agitation 0,800 l de Cétavlon® à 3%. Après une heure d'agitation, on centrifuge à 10.000 t/mn, pendant 15 minutes. On élimine le précipité, on ajoute au surnageant isolé, 1,5 l d'éthanol à 95°. Après une heure d'agitation, on centrifuge pendant 15 minutes à 10.000 t/mn. Le surnageant est éliminé et le précipité repris dans 0,500 l d'eau et dialysé pendant 48 heures dans des tubes Visking® contre de l'eau permutée à +4°C.

Au terme de cette dialyse, on lyophilise la solution et on obtient 6 g des glycoprotéines cherchées.

## Exemple 3:

On a préparé des comprimés répondant à la formule:

— glycoprotéines obtenues à l'exemple 1                5 mg
— excipient q. s. pour un comprimé terminé à       100 mg
    (Détail de l'excipient: lactose, amidon, talc, stéarate de magnésium).

## Exemple 4:

On a préparé une pommade répondant à la formule:

— glycoprotéines obtenues à l'exemple 2             200 mg
— excipient q.s.p.                          100 g

### Etude Pharmacologique

#### A) — Activité immunostimulante et mitogénique.

On administre par voie intraplantaire, à des lots de 10 souris, 40 μg du produit à tester et 40 μg de Sérum-albumine bovine par animal. On leur administre dix jours plus tard par voie intraveineuse, une dose non létale et non choquante de Sérum-albumine (100 μg par animal). Des animaux témoins ne recoivent que la Sérum-albumine lors de la première injection.

On mesure l'activité immunostimulante par l'augmentation de la réponse de choc anaphylactique à la Sérum-albumine, et l'activité mitogénique par l'augmentation du poids des ganglions drainant le point d'injection.

#### 1 — activité immunostimulantes:

On relève le nombre d'animaux présentant un état de choc (dyspnée avec cyanose du museau allant jusqu'à la paralysie du train arrière, les convulsions et la mort), ainsi que la mortalité, deux heures après l'injection intraveineuse de Sérum-albumine.

Résultats:

| Produit de exemple | % d'animaux choqués | % de morts |
|---|---|---|
| 1 | 50 | 20 |
| 2 | 50 | 30 |
| Témoin | 0 | 0 |

### 2 — activité mitogénique:

On sacrifie deux heures après l'injection intraveineuse de Sérum-albumine les survivants, prélève les ganglions poplités drainant la patte où a eu lieu l'injection et les pèse. L'activité mitogénique est exprimée à l'aide d'un index correspondant au rapport du poids moyen des ganglions des animaux traités avec le produit à tester, et de ceux des animaux témoins n'ayant reçu que la Sérum-albumine.

Résultats:

| Produit de l'exemple | Index |
|---|---|
| 1 | 6,2 |
| 2 | 5,6 |

Les produits des exemples 1 et 2 présentent donc de très bonnes activités immunostimulante et mitogénique.

### B) — Stimulation des défenses non spécifiques:

Cette stimulation a été étudiée sur le test de la »clearance« au carbone chez la souris en s'inspirant de la technique mise au point par HALPERN, qui consiste à injecter à l'animal dans le sinus oculaire, une suspension de carbone colloïdal, et à apprécier, en fonction du temps, la cinétique de la disparition du carbone dans le sang, en effectuant des mesures de densité optique.

Les produits sont administrés à l'animal par voie intrapéritonéale, vingt-quatre et quarante-huit heures avant le test. Les résultats sont exprimés en pourcentage d'élimination des particules de carbone par rapport à des témoins ayant reçu uniquement l'injection de carbone colloïdal et correspondant aux 100% du nombre de particules de carbone.

| Produit de l'exemple | Doses | % d'activité par rapport aux Témoins | |
|---|---|---|---|
| | | 8 minutes après l'injection | 30 minutes après l'injection |
| 1 | 0,25 mg/kg | 50% | 70% |
| 2 | 0,25 mg/kg | 60% | 70% |

L'examen de ces résultats nous permet de noter l'intense stimulation provoquée par ces deux produits, sur les défenses de l'organisme.

### C) — Toxicité aiguë

La dose létale 50 (DL 50) par voie intrapéritonéale, chez la souris, a été déterminée par la méthode de BEHRENS et KARBER.

Elle est de 50 mg/kg pour les glycoprotéines des exemples 1 et 2.


D) — Tolérance

L'injection sous cutanée de 0,2 ml de glycoprotéines des exemples 1 et 2, à la dose de 1000 $\gamma$/kg, chez la souris, ne provoque aucune intolérance locale ou générale.

La souche déposée à l'Institut Pasteur sous le n° 52 145 a été redédosée à l'Institut Pasteur le 25 Juin 1981 sous le n° I-163.


## Revendications pour les Etats contractants: BE, CH, DE, GB, IT, LI, LU, NL, SE

1. Glycoprotéines hydrosolubles immunostimulantes extraites de Klebsiella pneumoniae, caractérisées en ce qu'elles renferment 20% à 35% de protéines, 50% à 70% d'oses neutres, 3% à 8% d'acide glucuronique, 1,5% à 3% d'osamines et ont un poids moléculaire supérieur à 100.000 daltons.

2. Nouvelles glycoprotéines selon la revendication 1, caractérisées en ce qu'elles sont extraites de la souche déposée à l'Institut PASTEUR, seus le n° 52.145.

3. Nouvelles glycoproteines, selon la revendication 1 ou 2, caractérisées en ce que la fraction protéique est composée par environ 30% d'acides aminés acides.

4. Nouvelles glycoprotéines selon l'une quelconque des revendications 1 à 3, caractérisées en ce que la fraction polysaccharidique renferme approximativement pour 1 molécule de glucose, de 1 à 1,5 molécule de mannose, de 0,2 à 0,6 molécule d'acide glucuronique et de 2 à 2,5 molécules de galactose.

5. Procédé d'obtention des nouvelles glycoprotéines telles que définies à l'une quelconque des revendications 1 à 4, dans lequel l'on prépare un extrait de glycoprotéines obtenu par mise en culture de germes de Klebsiella pneumoniae, lyse des germes, sèchage, délipidation par extraction à l'aide de solvants des lipides, et ultrafiltration, ledit procédé étant caractérisé en ce que l'on traite la solution aqueuse de glycoprotéines ainsi obtenue à l'aide d'un sel d'ammonium quaternaire, obtient ainsi un précipité que l'on élimine, traite à froid le surnageant à l'aide d'un alcanol de faible poids moléculaire, obtient ainsi un nouveau précipité que l'on isole, redissout dans l'eau, dialyse le cas échéant puis, lyophilise.

6. Procédé selon la revendication 5, caractérisé en ce que:

— l'ammonium quaternaire utilisé est le bromure de cétyltriméthyl-ammonium;
— l'on isole par centrifugation le surnageant;
— l'alcanol de faible poids moléculaire est l'éthanol.

7. Les nouvelles glycoprotéines telles qu'obtenues par le procédé selon la revendication 5 ou 6.

8. Médicaments, caractérisés en ce qu'ils sont constitués par les nouvelles glycoprotéines hydrosolubles telles que définies à l'une quelconque des revendications 1 à 4.

9. Médicaments, caractérisés en ce qu'ils sont constitués par les glycoprotéines telles que définies à la revendication 7.

10. Compositions pharmaceutiques, caractérisées en ce qu'elles renferment, à titre de principe actif, l'un au moins des médicaments tels que définis à la revendication 8 ou 9.


## Revendications pour l'Etat contractant: AT

1. Procédé de préparation de glycoprotéines hydrosolubles immunostimulantes extraites de Klebsiella pneumoniae renfermant 20% à 35% de protéines, 50% à 70% d'oses neutres, 3% à 8% d'acide glucuronique, 1,5% à 3% d'osamines et ayant un poids moléculaire supérieur à 100.000 daltons, dans lequel l'on prépare un extrait de glycoprotéines obtenu par mise en culture de germes de Klebsiella pneumoniae, lyse des germes, sèchage, délipidation par extraction à l'aide de solvants des lipides, et ultrafiltration, ledit procédé étant caractérisé en ce que l'on traite la solution aqueuse de glycoprotéines ainsi obtenue à l'aide d'un sel d'ammonium quaternaire, obtient ainsi un précipité que l'on élimine, traite à froid le surnageant à l'aide d'un alcanol de faible poids moléculaire, obtient ainsi un nouveau précipité que l'on isole, redissout dans l'eau, dialyse le cas échéant puis, lyophilise.

2. Procédé selon la revendication 1, caractérisé en ce que:

— l'ammonium quaternaire utilisé est le bromure de cétyltriméthyl-ammo nium.
— l'on isole par centrifugation le surnageant,
— l'alcanol de faible poids moléculaire est l'éthanol.

3. Procédé selon la revendication 1 ou 2, caractérisé en ce que les glycoprotéines sont extraites de la souche déposée à l'Institut PASTEUR, sous le n° 52.145.

4. Procédé selon la revendication 1, 2 ou 3, caractérisé en ce que la fraction protéique est composée par environ 30% d'acides aminés acides.

5. Procédé selon la revendication 1, 2, 3 ou 4, caractérisé en ce que la fraction polysaccharidique renferme aproximativement pour 1 molécule de glucose, de 1 à 1,5 molécule de mannose, de 0,2 à 0,6 molécule d'acide glucuronique et de 2 à 2,5 molécules de galactose.

## Patentansprüche für die Vertragsstaaten: BE, CH, DE, GB, IT, LI, LU, NL, SE

1. Aus Klebsiella pneumoniae extrahierte immunostimulierende wasserlösliche Glykoproteine, dadurch gekennzeichnet, daß sie 20 bis 35% Proteine, 50 bis 70% neutrale Osen, 3 bis 8% Glucuronsäure, 1,5 bis 3% Osamine umfassen und ein Molekulargewicht von höher als 100 000 Dalton besitzen.

2. Neue Glykoproteine gemäß Anspruch 1, dadurch gekennzeichnet, daß sie aus dem in dem Pasteur-Institut unter der Nummer 52.145 hinterlegten Stamm extrahiert sind.

3. Neue Glykoproteine gemäß Anspruch 1 oder 2, dadurch gekennzeichnet, daß die Proteinfraktion aus etwa 30% sauren Aminosäuren besteht.

4. Neue Glykoproteine gemäß einem der Ansprüche 1 bis 3, dadurch gekennzeichnet, daß die Polysaccharid-Fraktion je 1 Molekül Glucose etwa 1 bis 1,5 Moleküle Mannose, 0,2 bis 0,6 Moleküle Glucuronsäure und 2 bis 2,5 Moleküle Galactose umfaßt.

5. Verfahren zur Herstellung neuer Glykoproteine gemäß einem der Ansprüche 1 bis 4, bei dem man einen Glykoproteine-Extrakt herstellt, erhalten durch Kultivierung von Klebsiella pneumoniae-Keimen, Lyse der Keime, Trocknung, Befreiung von Lipiden bzw. Lipoiden durch Extraktion mit Lösungsmitteln für Lipide bzw. Lipoide und Ultrafiltration, dadurch gekennzeichnet, daß man die so erhaltene, wäßrige Glykoproteine-Lösung mit einem quaternären Ammoniumsalz behandelt, auf diese Weise einen Niederschlag erhält, den man eliminiert, die überstehende Flüssigkeit in der Kälte mit einem Alkanol mit niedrigem Molekulargewicht behandelt, so einen neuen Niederschlag erhält, den man isoliert, erneut in Wasser löst, gegebenenfalls dialysiert und danach lyophilisiert.

6. Verfahren gemäß Anspruch 5, dadurch gekennzeichnet, daß

das verwendete quaternäre Ammonium Cetyltrimethylammoniumbromid ist,
die überstehende Flüssigkeit durch Zentrifugation isoliert wird,
das Alkanol mit niedrigem Molekulargewicht Ethanol ist.

7. Neue Glykoproteine, erhalten durch ein Verfahren gemäß Anspruch 5 oder 6.

8. Arzneimittel, dadurch gekennzeichnet, daß sie aus den neuen, wasserlöslichen Glykoproteinen gemäß einem der Ansprüche 1 bis 4 bestehen.

9. Arzneimittel, dadurch gekennzeichnet, daß sie aus den Glykoproteinen gemäß Anspruch 7 bestehen.

10. Pharmazeutische Zusammensetzungen, dadurch gekennzeichnet, daß sie als Wirkstoff zumindest eines der Arzneimittel gemäß Anspruch 8 oder 9 enthalten.

## Patentansprüche für den Vertragsstaat: AT

1. Verfahren zur Herstellung aus Klebsiella pneumoniae extrahierter immunostimulierender wasserlöslicher Glykoproteine, umfassend 20 bis 35% Proteine, 50 bis 70% neutrale Osen, 3 bis 8% Glucuronsäure, 1,5 bis 3% Osamine, mit einem Molekulargewicht von größer als 100 000 Dalton, bei dem man einen Glykoproteine-Extrakt herstellt, erhalten durch Kultivierung von Klebsiella pneumoniae-Keimen, Lyse der Keime, Trocknung, Befreiung von Lipiden bzw. Lipoiden durch Extraktion mit Lösungsmitteln für Lipide bzw. Lipoide und Ultrafiltration, dadurch gekennzeichnet, daß man die so erhaltene, wäßrige Glykoproteine-Lösung mit einem quaternären Ammoniumsalz behandelt, auf diese Weise einen Niederschlag erhält, den man eliminiert, die überstehende Flüssigkeit in der Kälte mit einem Alkanol mit niedrigem Molekulargewicht behandelt und so einen neuen Niederschlag erhält, den man isoliert, erneut in Wasser löst, gegebenenfalls dialisiert und danach lyophilisiert.

2. Verfahren gemäß Anspruch 1, dadurch gekennzeichnet, daß

das verwendete quaternäre Ammonium Cetyltrimethylammoniumbromid ist,
man die überstehende Flüssigkeit durch Zentrifugation isoliert,
das Alkanol mit niedrigem Molekulargewicht Ethanol ist.

3. Verfahren gemäß Anspruch 1 oder 2, dadurch gekennzeichnet, daß die Glykoproteine aus dem in dem Pasteur-Institut unter der Nummer 52.145 hinterlegten Stamm extrahiert werden.

4. Verfahren gemäß Anspruch 1, 2 oder 3, dadurch gekennzeichnet, daß die Proteinfraktion aus etwa 30% sauren Aminosäuren besteht.

5. Verfahren gemäß Anspruch 1, 2, 3 oder 4, dadurch gekennzeichnet, daß die Polysaccharid-Fraktion je 1 Molekül Glucose etwa 1 bis 1,5 Moleküle Mannose, 0,2 bis 0,6 Moleküle Glucuronsäure und 2 bis 2,5 Moleküle Galactose umfaßt.

## Claims for the contracting states: BE, CH, DE, GB, IT, LI, LU, NL, SE

1. Immunostimulating hydrosoluble glycoproteins extracted from Klebsiella pneumoniae, characterized in that they contain 20%—35% of proteins, 50% to 70% of neutral carbohydrates, 3% to 8% of glucuronic acid, 1.5% to 3% of osamines and have a molecular weight greater than 100,000 daltons.

2. New glycoproteins according to Claim 1, characterized in that they are extracted from the strain filed at the PASTEUR Institute, under number 52,145.

3. New glycoproteins, according to Claim 1 or 2, characterized in that the protein fraction is composed of about 30% of acid amino acids.

4. New glycoproteins according to any one of Claims 1 to 3, characterized in that the polysaccharide fraction contains approximately 1 to 1.5 molecules of mannose, 0.2 to 0.6 molecules of glucuronic acid and 2 to 2.5 molecules of galactose per 1 molecule of glucose.

5. Process for obtaining new glycoproteins as defined in any one of Claims 1 to 4, in which a glycoprotein extract is obtained by culturing germs of Klebsiella pneumoniae, lysing the germs, drying, removal of lipids by extracting with lipid solvents, and ultrafiltration, the said process being characterized in that the aqueous solution of glycoproteins thus obtained is treated with a quaternary ammonium salt, a precipitate obtained in this way is eliminated, the supernatant is treated when cold with an alkanol of low molecular weight, a new precipitate thus obtained is isolated, dissolved in water again, dialyzed if applicable, then lyophilized.

6. Process according to Claim 5, characterized in that

— the quaternary ammonium utilized is the bromide of cetyltrimethyl-ammonium;
— the supernatant is isolated by centrifuging;
— the alkanol of low molecular weight is ethanol.

7. New glycoproteins as obtained by the process according to Claim 5 or 6.

8. Medicaments, characterized in that they are constituted by new hydrosoluble glycoproteins as defined in any one of Claims 1—4.

9. Medicaments, characterized in that they are constituted by the glycoproteins as defined in Claim 7.

10. Pharmaceutical compositions, characterized in that they contain, as active principle, at least one of the medicaments as defined in Claim 8 or 9.

## Claims for the contracting state: AT

1. Preparation process for immunostimulating, hydrosoluble glycoproteins extracted from Klebsiella pneumoniae, containing 20% to 30% of proteins, 50% to 70% of neutral carbohydrates, 3% to 8% of glucuronic acid, 1.5% to 3% of osamines and having a molecular weight greater than 100,000 daltons, in which a glycoprotein extract is prepared by culturing germs of Klebsiella pneumoniae, lysing the germs, drying, removal of lipids by extracting with lipid solvents, and ultrafiltering, the said process being characterized in that the aqueous solution of glycoproteins thus obtained is treated with a quaternary ammonia salt, a precipitate obtained in this way is then eliminated, the supernatant is treated when cold with an alkanol of low molecular weight, a new precipitate thus obtained is isolated, dissolved in water again, dialyzed if applicable, then lyophilized.

2. Process according to Claim 1, characterized in that

— the quaternary ammonia utilized is the bromide of cetyltrimethyl-ammonia,
— the supernatant is isolated by centrifuging,
— the alkanol of low molecular weight is ethanol.

3. Process according to Claim 1 or 2, characterized in that the glycoproteins are extracts of the strain filed at the PASTEUR Institute, under no. 52,145.

4. Process according to Claim 1, 2 or 3, characterized in that the protein fraction is composed of about 30% of acid amino acids.

5. Process according to Claim 1, 2, 3 or 4, characterized in that the polysaccharide fraction contains approximately 1—1.5 molecules of mannose, 0.2—0.6 molecules of glucuronic acid and 2—2.5 molecules of galactose per 1 molecule of glucose.